# EUROPÄISCHE PATENTSCHRIFT

(11) **EP 1 270 734 B1**
(45) Veröffentlichungstag und Bekanntmachung des Hinweises auf die Patenterteilung: **12.12.2007**
(21) Anmeldenummer: 01115081.0
(22) Anmeldetag: 21.06.2001
(51) Int. Cl.: C12P 7/20, C12P 7/40, C12P 7/64

(54) **Verfahren zur enzymatischen Spaltung von Ölen und Fetten**
Process for the enzymatic cleavage of oils and fats
Procédé pour le clivage des huiles et des matières grasses par fermentation

(43) Veröffentlichungstag der Anmeldung: 02.01.2003
(73) Patentinhaber: T + T Oleochemie GmbH, 63755 Alzenau (DE)
(72) Erfinder: Brunner, Karlheinz, Dr., 63538 Grosskrotzenburg (DE); Frische, Rainer, Dr., 60529 Frankfurt (DE); Kilian, Dirk, Dr., 63477 Maintal (DE)
(74) Vertreter: Englaender, Klaus

(56) Entgegenhaltungen:
- DE-A- 19 922 882
- DATABASE BIOSIS [Online] BIOSCIENCES INFORMATION SERVICE, PHILADELPHIA, PA, US; 1993 TANIGAKI MASANOBU ET AL: "Hydrolysis of soybean oil by lipase with a bioreactor having two different membranes." Database accession no. PREV199395084843 XP002182853 & JOURNAL OF FERMENTATION AND BIOENGINEERING, Bd. 75, Nr. 1, 1993, Seiten 53-57, ISSN: 0922-338X
- DATABASE BIOSIS [Online] BIOSCIENCES INFORMATION SERVICE, PHILADELPHIA, PA, US; 2. Dezember 1998 (1998-12-02) GAN Q ET AL: "Simultaneous reaction and separation in enzymatic hydrolysis of high oleate sunflower oil - evaluation of ultrafiltration performance and process synergy." Database accession no. PREV199900056287 XP002182854 & CHEMICAL ENGINEERING JOURNAL, Bd. 71, Nr. 2, 2. Dezember 1998 (1998-12-02), Seiten 87-96, ISSN: 0923-0467

## Beschreibung

Die Erfindung betrifft die enzymatische Spaltung nativer Öle und Fette, d.h. der Triglyceride der Öle und Fette, mit Wasser in Glycerin und Fettsäuren.

Diese Art der Spaltung ist seit langem bekannt und bietet gegenüber der in der Industrie hauptsächlich eingesetzten Druckspaltung beträchtliche grundsätzliche Vorteile. So kann die enzymatische Spaltung bei Normaldruck und (je nach Enzym und Öl bzw.Fett) selbst bei Raumtemperatur durchgeführt werden. Seit langem ist auch bekannt, daß dieser Weg der Fettspaltung der schonendste ist. Durch die Fortschritte bei der industriellen Biotechnologie sind auch für die Spaltung geeignete Enzyme verfügbar. Sie werden z.B. in zahlreichen Waschmitteln eingesetzt. Hauptsächlich wegen des hohen Enzymverbrauchs, der sehr langen Spaltzeiten und hieraus resultierender mangelnder Wirtschaftlichkeit hat sich dennoch die enzymatische Spaltung großtechnisch bisher nicht gegen die Druckspaltung durchgesetzt.

Die Hauptprobleme, die inzwischen bei den zahlreichen Versuchen der Absenkung des Enzymverbrauchs aufgetreten sind, sind aus den folgenden Veröffentlichungen ersichtlich: "Continuous Use of Lipases in Fat Hydrolysis", M. Bühler and Chr. Wandrey, Fat Science Technology 89/ Dez. 87, Seiten 598 bis 605; "Enzymatische Fettspaltung", M. Bühler and Chr. Wandrey, Fett Wissenschaft Technologie 89 / Nr.4 / 1987, Seiten 156 bis 164; und "Oleochemicals by Biochemical Reactions ?", M. Bühler and Chr. Wandrey, Fat Science Technology 94 / Nr. 3 / 1992, Seiten 82 bis 94.

In diesen Veröffentlichungen ist die enzymatische Öl- bzw. Fettspaltung unter Verwendung von Enzymen, sogenannten Lipasen, beschrieben, die als Biokatalysatoren auf ein Wasser/Öl Gemisch einwirken. Dabei wird das Öl/Fett in Glycerin und freie Fettsäuren gespalten. Das Glycerin wandert dabei in die Wasserphase, die organische Phase reichert sich mehr und mehr an freien Fettsäuren an, bis letztlich lediglich freie Fettsäuren als organische Phase verbleiben.

Allerdings nimmt die Aktivität des eingesetzten Enzyms, weitgehend unabhängig von der Menge an katalytisch umgesetztem Produkt, im Verlaufe der Zeit ab. Diese Abnahme kann zwar durch Nachdosierung kompensiert werden, ein zeitabhängig variierender Enzymverbrauch ist jedoch nicht vermeidbar. Im Verlaufe der Spaltreaktion nimmt die Reaktionsgeschwindigkeit immer stärker ab und der Enzymverbrauch nimmt damit zu. Dies liegt daran, daß die enzymatische katalysierte Hydrolyse eine Gleichgewichtsreaktion ist. Mit zunehmender Konzentration an Glycerin in der Wasserphase und Fettsäure in der organischen Phase wird die Reaktionsgeschwindigkeit verlangsamt, um sich letztlich asymptotisch der Gleichgewichtskonzentration zu nähern. Die anzustrebenden Spaltgrade nahe 100 % lassen sich daher nur nach sehr langer Reaktionszeit realisieren. Diese lange Reaktionszeit hat zwangsweise einen hohen Aktivitätsverlust an Enzym zur Folge. Die Reaktionszeit läßt sich zwar durch Absenkung der Glycerinkonzentration in der Wasserphase verkürzen. Dies hat jedoch zur Folge, daß das gewonnene Spaltglycerin in niedriger Konzentration anfällt und aufgrund des höheren Anteils an Wasserphase mehr gelöstes Enzym enthält, das dann mit der Wasserphase ausgeschleust wird und nicht wieder eingesetzt werden kann.

Die enzymatische Spaltreaktion findet an der Phasengrenze zwischen organischer und wässriger Phase statt, wobei lediglich das in der Phasengrenze vorhandene Enzym und die an der Phasengrenze vorhandenen Triglyceride spaltwirksam sind. Mit zunehmendem Spaltgrad nimmt die Belegungsdichte an der Phasengrenze an glyceridisch gebundenen Fettsäuren im Vergleich zu freien Fettsäuren ab, so daß die Reaktion verlangsamt wird. Zwar kann durch Vergrößerung der Phasengrenzfläche die Reaktionsgeschwindigkeit beschleunigt werden, dies verlangt jedoch, die Enzymmenge zu erhöhen, damit die Belegungsdichte an Enzym an der Grenzfläche gleich bleibt. Die Erhöhung der Reaktionsgeschwindigkeit durch Zugabe von Enzym ist allerdings begrenzt. Über einem Belegungsdichtemaximum wirken weitere Zugaben an Enzym nicht mehr reaktionsbeschleunigend. Der Enzymverbrauch wird hierdurch allerdings spürbar erhöht, so daß eine optimale Einstellung der Enzymmenge und Größe der Phasengrenzfläche nicht einfach zu erzielen ist.

Bei der Trennung der organischen Fettsäurephase und der Wasserphase werden ferner zwangsweise nicht wieder verwendbare Enzymmengen ausgeschleust und der Wiederverwendung entzogen. Zwar kann die Reaktionsgeschwindigkeit durch Erhöhung Phasengrenzfläche (durch intensive Durchmischung der organischen und wässrigen Phase) und der Enzymmenge erhöht werden, die Phasenabtrennung, Rückgewinnung und Wiederverwendung des Enzyms werden dadurch jedoch erschwert.

In den genannten Veröffentlichungen erfolgt die enzymatische Spaltreaktion im Gegenstrom von Wasser und zu spaltendem Öl mehrstufig und kontinuierlich. Bei der Trennung der Glycerin haltigen wässrigen Phase und der die abgespaltenen freien Fettsäuren enthaltenden organische Phase entsteht eine Zwischenschicht. Diese enthält den größten Anteil an Enzym. Um dieses Enzym für den Prozeß zu erhalten und den Enzymverbrauch zu verringern, wird gemäß der obigen Veröffentlichung "Continuous Use of Lipases in Fat Hydrolysis" folgendermaßen vorgegangen: Öl wird in einem ersten Rühr-Reaktor kontinuierlich gespalten. Das Reaktionsprodukt, das neben freien Fettsäuren Wasser, Glycerin und Mono- und Diglyceride, ungespaltenes Öl sowie Enzym enthält, wird in eine Vollmantel-Tellerzentrifuge gegeben. Diese wird so eingestellt, daß die Zwischenschicht zwischen wässriger Glycerinphase und organischer Phase mit der organischen Phase ausgetragen wird. Die die Zwischenschicht enhaltende organische Phase wird einem zweiten Rühr-Reaktor zugeführt, dem ferner eine frische Wasser/Enzymmischung zugeführt wird. Das Reaktionsprodukt des zweiten Reaktors wird wiederum einer Vollmantel-Tellerzentrifuge zugeführt, die allerdings nun so eingestellt wird, daß die Zwischenschicht mit der glycerinhaltigen wässrigen Phase und die entstehenden freien Fettsäuren ohne Emulsions-Zwischenschicht ausgeschleust werden. Die wässrige Phase wird in den ersten Reaktor zurückgeleitet, so daß dort die in der Emulsions-Zwischenschicht enthaltenen Enzymmengen dem Prozeß erneut zugeführt werden. Der im zweiten Reaktor erzielte Spaltgrad beträgt bis zu 98%, so daß die Ausbeute an freien Fettsäuren nach Destillation des End-Reaktionspodukts nach dem zweiten Reaktor beachtlich ist. Allerdings ist auch bei dieser Reaktionsführung immer noch kein zur großtechnisch betriebenen Druckspaltung konkurrenzfähiger Prozeß möglich.

Der vorliegenden Erfindung liegt die Aufgabe zugrunde, das Verfahren großtechnisch rentabel zu machen.

Diese Aufgabe wird durch Anspruch 1 gelöst. Vorteilhafte Weiterbildungen sind in den Unteransprüchen definiert. Die erfindungsgemäßen Merkmale erbringen kurze Reaktionszeiten und einen verringerten Enzymverbrauch.

Bei den umfangreichen Versuchen zur Lösung dieser Aufgabe ermittelten die Erfinder der vorliegenden Anmeldung, daß von der in den oben erwähnten Veröffentlichungen aufgezeigten Verfahrensweise grundsätzlich in mehrfacher Hinsicht abgewichen werden muß.

So wird nach einem Lösungsaspekt der vorliegenden Erfindung davon abgegangen, bereits mit der enzymatischen Spaltung die gewünschte Ausbeute der freien Fettsäuren dadurch zu bewerkstelligen, daß die enzymatische Spaltung bis zu einem hohen Spaltgrad betrieben wird. Stattdessen wird die enzymatische Spaltung in der vorliegenden Erfindung nur bis zu einem vergleichsweise geringen Spaltgrad betrieben, und zwar bis zu einem Spaltgrad, bei dem noch keine nennenswerte Verlangsamung des Prozesses stattfindet. Ein solcher Spaltgrad liegt in der Regel zwischen etwa 80% bis 90% und damit unter den Spaltgradwerten, die man bislang als unerläßlich für einen kommerziell anwendbaren Prozeß angesehen hat. Aus der vom Enzym befreiten, organischen, Fettsäure haltigen Phase werden die freien Fettsäuren abgetrennt und der die noch glyceridisch gebundenen Fettsäuren enthaltende Rest wird zurückgeführt und mit frischem, zu spaltendem Öl oder Fett vermischt und enzymatisch nachgespalten.

Betreibt man die enzymatische Fettspaltung lediglich bis zu einem Spaltgrad von etwa 80%, so ist dies in sehr kurzer Zeit möglich. Entfernt man anschließend aus einem derart teilgespaltenen Öl oder Fett die freien Fettsäuren und führt die glyceridisch gebundenen Fettsäuren in die Spaltung zurück, so läßt sich der Enzymverbrauch drastisch verringern, ohne auf eine vollständige Spaltung des Öls verzichten zu müssen. Der enzymatische Spaltprozeß gelangt nicht in den kritischen Bereich, in dem die Reaktionsgeschwindigkeit durch mangelnde Verfügbarkeit von glyceridischen Fettsäuren an der Grenzphase deutlich verlangsamt wird.

Zur Abtrennung der freien Fettsäuren aus dem teilgespaltenen Ausgangsprodukt der enzymatischen Spaltung wird vorzugsweise die Vakuumdestillation angewandt, und zwar bevorzugt eine schonende Kurzwegdestillation. Bei Fetten und Ölen mit einem Kettenlängenspektrum der Fettsäuren von C14 bis C22, wie es für die überwiegende Anzahl von natürlichen Fetten und Ölen üblich sind, können die freien Fettsäuren problemlos destillativ aus dem teilgespaltenen Ausgangsprodukt, d.h. Fett oder Öl, entfernt werden, ohne daß nennenswerte Mengen an glyceridisch gebundenen Fettsäuren mit abdestilliert werden. So sind bei Spaltgraden zwischen etwa 80 und 90 % überraschender Weise die Anteile an mono- und diglyceridisch gebundenen Fettsäuren gering. Der Hauptteil besteht aus noch nicht gespaltenen Triglyceriden. Im Rückstand verbleibende Restmengen an freien Fettsäuren führen zu keinem Verlust, da der Rückstand als der verbleibende Rest zur Spaltreaktion zurückgeführt wird.

Alternativ zur destillativen Abtrennung der freien Fettsäuren kann eine adsorptive Trennung (z.B. Säulenchromatographie) eingesetzt werden.

Vorteilhaft werden zur destillativen Ausschleusung der gebildeten freien Fettsäuen durch Vakuumdestillation Vakuum-Dünnfilm-Verdampfer wie Fallfilmverdampfer oder Kurzwegdestillen eingesetzt, da diese Destillationstechniken im Vergleich zur Blasendestillationstechnik schonender sind und vor allem kontinuierlich betrieben werden können. Zudem verlangen diese Destillationstechniken ohnehin einen flüssigen Restrückstand von ca. 5% bis 10%, da andernfalls der Destillationsfilm reißt. Daher ist es hier von erheblichem Vorteil, wenn erfmdungsgemäß ein Reaktionsprodukt mit noch relativ geringem Spaltgrad zugeführt wird. Vor der Rückführung des aus der Destille ablaufenden Sumpfes werden bevorzugt durch bekannte chemisch-physikalische Maßnahmen, wie z.B. Entwachsen, Winterisieren und Bleichen, Wachse oder andere unerwünschte Begleitstoffe ausgeschleust, die sich sonst bei der Rückführung anreichern würden.

Durch die obigen Maßnahmen konnte z.B. bei der Spaltung von High-Oleic-Sonnenblumenöl die Reaktionszeit drastisch verkürzt werden, die sonst erforderlich gewesen wäre, um bei der enzymatischen Spaltung einen Spaltgrad von 98 % zu erreichen. Darüber hinaus führte der frühzeitige Abbruch der enzymatischen Spaltung auch dazu, daß die Enzymverluste, die auf die zeitliche Aktivitätsabnahme des Enzyms zurückgehen, entsprechend stark auf einen Bruchteil des Verlustes gesenkt wurden, der bei einem Arbeiten bis zum Spaltgrad von 98% aufgetreten wäre. Somit ist das derart ausgebildete Verfahren der enzymatischen Fettspaltung der Druckspaltung kostenmäßig überlegen. Hiermit bietet das Verfahren nach dem Gegenstand des Anspruchs 1 erstmalig die Möglichkeit, die enzymatische Spaltung sogar mit Vorteil alternativ zur bisherigen Druckspaltung in der industriellen Anwendung einzusetzen.

Nach einem weiteren von den Erfindern ermittelten Lösungsaspekt können die Prozeßzeit und der Enzymverbrauch dadurch erheblich verbessert werden, daß für zur Trennung von schwerer wässriger Phase und leichter, die abgetrennten Fettsäuren enthaltender organischer Phase ein selbstaustragender Separator (selbstaustragende Zentrifuge mit Tellerpaket) eingesetzt wird, der so eingestellt wird, daß weder die organische Phase noch die wässrige Phase nennenswerte Mengen enzymhaltiger Phasengrenzemulsion enthalten, sondern sich diese im Separator im Bereich der im Tellerpaket liegenden Trennzone anreichern. Dies stellt eine insofern ungewöhnliche Einstellung dar, als daß man auch bei selbstaustragenden Separatoren, den sogenannten selbst entschlammenden Separatoren, üblicherweise gerade vermeidet, daß sich bei einer Flüssig/Flüssigphasentrennung größere Mengen einer sich bildenden Zwischenschicht im Tellerpaket ansammeln. Stattdessen sorgt man im allgemeinen dafür, daß diese Zwischenschicht möglichst gering ausfällt und in der nicht primär zu gewinnenden Phase mit ausgeschleust wird. So wurde im übrigen prinzipiell auch in der obigen Veröffentlichung vorgegangen, in der die Zwischenschicht hinter der zweiten kontinuierlich arbeitenden Mantelzentrifuge mit der wässrigen Phase ausgeschleust wurde und zum ersten Reaktor zurückgeleitet wurde.

Die erfindungsgemäß im Tellerpaket des Separators angesammelte emulsionsartige Zwischenschicht wird diskontinuierlich durch periodische Vollentleerungen der Trommel ausgetragen und die ausgetragene, Enzym enthaltende Zwischenschicht wird wiederverwendet. Dabei ist es zweckmäßig, die Trommelentleerung immer dann vorzunehmen, wenn die ausgeschleuste organische Phase beginnt, sich mit ausgeschleuster Zwischenschicht einzutrüben. Die Tatsache, daß bei der Vollentleerung auch wässrige Phase und organische Phase mit ausgetragen werden, ist nicht nachteilig, da sämtliche Phasen durch Rückführung in den Reaktor wiederverwendet werden.

Auf diese Weise werden weder über die schwere, wässrige Phase noch über die leichte, organische fettsäurehaltige Phase während des Trennprozesses nennenswerte Enzymmengen ausgeschleust und verloren. Der weitaus größte Teil der Enzymmenge kumuliert im Tellerpaket des Separators und kann durch die diskontinuierlich durchgeführte Teil- bzw. Vollentleerung zurückgewonnen und erneut verwendet werden. Statt einer Vollentleerung sind auch Teilentleerungen anwendbar, die allerdings so einzustellen sind, daß die Zwischenschicht möglichst vollständig ausgeschleust wird.

Auf die dargelegte Weise lassen sich die Verluste an mit der wässrigen Phase und organischen Phase ausgetragenem Enzym drastisch senken. Im Vergleich zum zeitabhängigen Enzymverbrauch (Enzymalterung) sind die Ausschleusungsverluste bei dieser erfindungsgemäßen Verfahrensweise sehr gering.

Eine technisch interessante Ergänzung der Erfindung zur weiteren Herabsetzung eines Enzymverlustes in der abgetrennten organischen Phase besteht in der Verwendung eines zusätzlichen selbstaustragenden Polierseparators. Dieser zusätzliche Separator wird unmittelbar hinter dem Separator der einzigen oder letzten Spaltstufe vorgesehen und erhält von diesem die ausgeschleuste organische Phase. Es handelt sich bei diesem zusätzlichen Separator vorzugsweise um eine selbstaustragende Zentrifuge mit Tellerpaket, die so eingestellt wird, daß sich die sedimentierenden Feststoffe, d.h. hier die Enzyme, und die noch abschleuderbaren Reste an wässriger Phase an der Trommelwand abscheiden. Die so abgeschleuderten Enzymmengen werden dann wiederum diskontinuierlich ausgetragen und in den Spaltprozeß zurückgeführt.

Vorteilhaft läßt sich vor allem bei Verwendung eines selbstaustragenden Separators zur Abtrennung der fettsäurehaltigen organischen Phase die Spaltreaktion in Schlaufenreaktoren diskontinuierlich, oder mit anderen Worten, absatz- oder batchweise durchführen und nicht kontinuierlich und in Durchlaufreaktoren, wie in den genannten Veröffentlichungen. So wird z.B. ein Reaktor mit Öl befüllt, wobei dessen Schlaufe für die Umwälzung des Reaktorinhalts aus Öl bzw. Fett, Wasser und Enzym nicht aktiv ist. Ein weiterer Reaktor führt gleichzeitig die Spaltreaktion durch mit aktiver Schlaufe und ein dritter Reaktor wird während dieser Zeit über einen selbstaustragenden Separator abgefahren, wobei das Reaktionsgemisch in wässrige Glycerin haltige Phase, organische Phase mit abgetrennten Fettsäuren und enzymhaltige Emulsionsgrenzphase, die sich als Zwischenschicht bildet, getrennt wird. Die Emulsionsgrenzphase wird von Zeit zu Zeit diskontinuierlich aus dem Separator ausgetragen, durch Versetzen mit frischem Enzym nachgeschärft und dem Reaktor wieder zugeführt.

Die Spaltreaktion kann so auf sehr hohem Enzymniveau gefahren werden, da durch die Umwälzung und die in den Umwälzpumpen zur Wirkung kommenden Scherfelder des Schlaufenreaktorbetriebs eine außerordentlich große Phasengrenzfläche entsteht. Wenn entsprechend dem ersten Aspekt der vorliegenden Erfindung lediglich z.B. nur ein 80 bis 90%iger Spaltgrad erzielt werden soll, kann zudem die zugeführte Wassermenge wesentlich verringert werden und eine wässrige Phase mit wesentlich erhöhter Glycerinkonzentration gewonnen werden, als dies zuvor möglich war. Selbst bei über 30 % Glycerin in der ausgeschleusten wässrigen Phase findet in diesem Fall keine nennenswerte Verlängerung der Reaktionszeit statt. Derartig hohe Glycerinkonzentrationen hatte man bislang nicht für praktikabel gehalten.

An dieser Stelle sei darauf hingewiesen, daß es durch die Betriebsweise nach diesem dritten Aspekt der vorliegenden Erfindung gelang, entgegen dem aufgezeigten Stand der Technik in einem kurzen Zeitrahmen von nur einer Stunde bereits einen Spaltgrad von bis zu 93% zu erzielen. Auch hier läßt sich noch ohne weiteres eine einstufige Kurzwegdestillation gemäß dem ersten Aspekt der vorliegenden Erfindung durchführen. Bei einem Spaltgrad von 95% oder mehr wäre dies allerdings aus den oben bereits erwähnten Gründen sehr erschwert.

Dadurch, daß erfindungsgemäß gemäß Anspruch 1 bei der Destillation der nicht abdestillierte Rest oder Sumpf in die Spaltung zurückgeführt wird, wird das Material vollständig umgesetzt, ohne, wie sonst üblich, mehrere Destillationsstufen oder andere Abtrennstufen für die Fettsäuren hintereinander zu schalten.

Besonders vorteilhaft läßt sich die erfindungsgemäße diskontinuierliche Spaltreaktion mit Umwälzreaktoren mehrstufig, z.B. in zwei Stufen durchführen, d.h. mit zwei Fettspaltungsstufen. Dabei wird bevorzugt ca. zwei Drittel der Spaltleistung in der ersten Stufe erzielt, in der die wässrige Glycerin haltige Phase der zweiten Stufe als Wasserphase eingesetzt wird, und ein Drittel der erwünschten Spaltung wird in der zweiten Stufe durchgeführt, der als Wasserphase Frischwasser zugeführt wird. Auf diese Weise lassen sich auch Spaltgrade von mehr als 90 % erzielen, ohne die Reaktionszeit nennenswert zu verlängern. Beide Stufen haben dabei eine eigene Kreislaufführung des batchweise, in Intervallen ausgetragenen Enzyms, wie weiter oben erwähnt. Das in den jeweiligen Prozessstufen kreislaufgeführte Enzym wird hauptsächlich zur Kompensation der Enzymalterung mit frischem Enzym nachgeschärft. Die Verluste durch Ausschleusen mit der wässrigen und organischen Phase fallen erfindungsgemäß jedoch nicht ins Gewicht.

Besonders vorteilhaft verläuft der Spaltprozeß, wenn man alle drei erfmdungsgemäßen Lösungsaspekte kombiniert, die aber bereits jeweils für sich allein die gestellte Aufgabe lösen. Durch die Kombination konnte die Reaktionszeit für eine vollständige Umsetzung ohne weitere Prozeßoptimierung unter zwei Stunden gedrückt werden und der Verbrauch an Enzym konnte auf 0,02 Gew.% bezogen auf das eingesetzte Öl reduziert werden. Dieser Wert konnte durch den Einsatz des nachgeschalteten Polierseparators weiter gesenkt werden.

Das erfindungsgemäße Verfahren der enzymatischen Fett/Ölspaltung ist erstmals auch bei großtechnischer Anwendung der Hochdruckspaltung überlegen: die Enzymverbrauchskosten liegen deutlich unter den Abschreibungskosten für den Investitionsmehraufwand einer Druckspaltung, die Enzymspaltung liefert qualitativ entschieden hochwertigere Produkte, der Energieverbrauch ist deutlich niedriger und das Verfahren ist sicherheitstechnisch unproblematisch. Die Produkte sind insbesondere deshalb hochwertiger, weil sie aufgrund der geringen Temperaturbelastung weniger Nebenprodukte aufweisen (Zersetzungsprodukte und Umlagerungsprodukte, wie sie z.B. bei Ölen mit ungesättigten Fettsäuren auftreten). Mit den erfindungsgemäßen Merkmalen kann das Material allein durch die enzymatische Spaltung ohne Nachschaltung einer Druckspaltung vollständig gespalten werden.

Bevorzugt werden erfindungsgemäß wie in den obigen Veröffentlichungen unspezifische oder Mischungen aus unspezifischen und spezifischen Lipasen als Enzyme eingesetzt. Ein geeignetes Enzym ist im Ausführungsbeispiel angegeben.

Alternativ kann man den erfindungsgemäßen Prozeß nach jedem der Lösungsaspekte auch nur mit spezifischen Enzymen fahren und so gezielt z.B. Monoglycerinoleat oder andere Mono- oder Diglyceride erzeugen. In diesem Fall wird man versuchen, das Monoglycerinoleat im Rückstand nach der Destillation zu maximieren, was mit den erfindungsgemäßen Merkmalen durchaus möglich ist.

Das erfindungsgemäße Verfahren ist auch dazu geeignet, die Mono-, Di- und Triglyceride zu spalten, die in den sogenannten Soap-Stocks aus der Alkali-Raffination von Speiseölen enthalten sind. Man kann so den Soap-Stock ohne vorhergehende Verseifung des Neutralöles quantitativ zu Fettsäuren umsetzen. Hierzu setzt man vorzugsweise vor der Spaltung durch Zugabe von Säure die in den Seifen gebundenen Fettsäuren frei.

Die Erfindung wird nun anhand eines bevorzugten Ausführungsbeispiels und der beiliegenden Zeichungen erläutert, wobei
FIG. 1 ein schematische Darstellung eines Beispiels für einen industriellen Prozess nach der Erfindung zeigt, und
FIG.2 bis 6 die bei den verschiedenen Prozeßstufen auftretenden Steigerungen der Säurezahl des eingesetzten Öles zeigen.

Im Technikumsversuch wurde mit rohem unraffiniertem High-Oleic Sonnenblumenöl gearbeitet. Wie zahlreiche vorausgehende Laborversuche zeigten, ist die Erfindung mit Vorteil auf praktische alle Öle und Fette, u.a. auch herkömmliches Sonnenblumenöl mit normalem Ölsäuregehalt anwendbar.

Bevorzugt wurde als wässrige Phase eine Pufferlösung aus Weichwasser (das frei von die Enzymreaktion behindernden Stoffen ist) und Natriumacetat eingesetzt. Es sind jedoch auch andere Puffersalze wie Natrium- oder Kaliumsalze, Carbonate, Citrate oder Phosphate möglich, um die Aktivität des Enzyms zu beschleunigen.

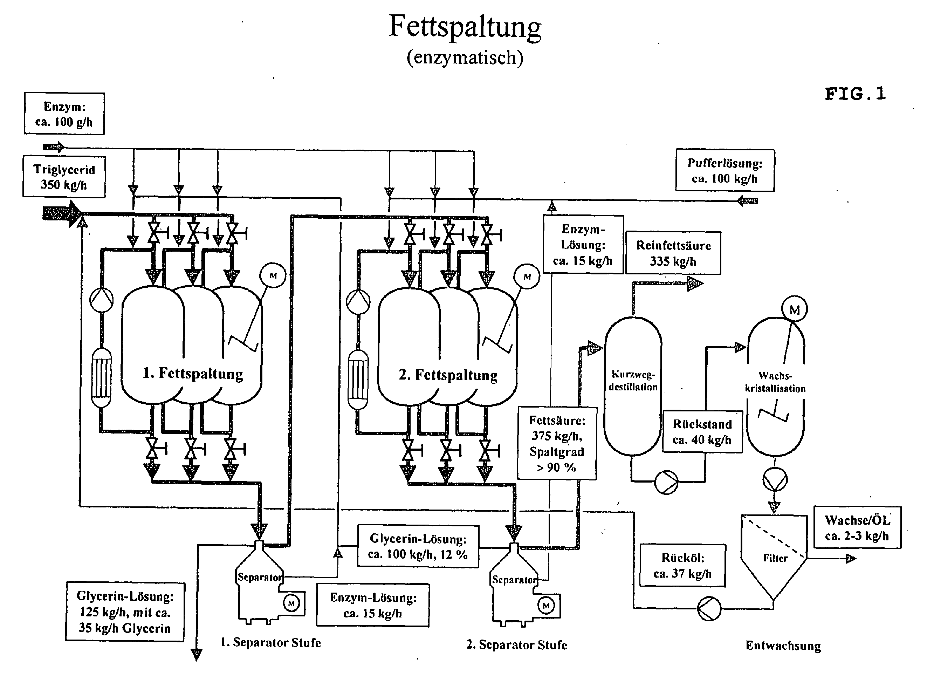

Die FIG. 1 zeig eine erfmdungsgemäße Anordnung zur zweistufen Fettspaltung, die dazu geeignet ist, die aufgezeigten Lösungsaspekte zu realisieren, wobei ein Beispiel für mögliche Mengenströme in einer industriellen Anlage angegeben sind. Es sind zwei Fettspaltungsstufen 1 und 2 vorgesehen, die jeweils drei Reaktoren mit Umwälzungsschlaufe umfassen. Die Reaktoren werden, wie weiter oben bereits dargelegt, intermittierend betrieben. Die jeweils für jeden der drei Reaktoren einer Stufe vorgesehene Umwälzungsschlaufe ist mit einer in der Zeichnung angedeuteten Kreiselpumpe und mit einem vorgeschalteten Wärmeaustauscher realisiert. Die Reaktoren bestehen z.B. aus Edelstahlbehältern mit Rührwerk. Ferner ist für jede Stufe ein Separator vorgesehen, und zwar in Form eines selbstaustragenden Teller-Separators.

Der Ausgang des Separators der zweiten Spaltstufe, aus dem die organische Phase mit den Fettsäuren ausgeschleust wird, ist mit einer Vakuum-Kurzweg-Destille verbunden, in der eine Kurzwegdestillation zur Abtrennung der freien Fettsäuren erfolgt. Das Rückstandsprodukt der Destillation wird einem Kristallisator mit Rührwerk zugeführt, in dem eine Wachskristallisation erfolgt. Im Anschluß daran wird das Rückstandsöl, in dem Wachse und andere höher siedende Begleitstoffe als Feststoffe auskristallisiert sind, über eine Pumpe in eine Filtereinrichtung eingebracht und dort von diesen Begleitstoffen befreit. Das so gereinigte Öl wird dann zur Spaltung vor die erste Stufe zurückgeleitet.

Den Reaktoren werden eine Pufferlösung, das zu spaltende Triglycerid und Enzym zugeführt. Ferner wird Enzym-Lösung bei jeder intermittierend herbeigeführten Entleerung der Separatoren gewonnen und zurück in den gerade zu befüllenden Reaktor derselben Stufe geleitet, dem auch der jeweilige Separator zugeordnet ist. Somit bleibt das Enzym mit gleichzeitig ausgeschleusten Anteilen an freien Fettsäuren, noch nicht gespaltenen Triglyceriden usw. im Kreislauf einer Stufe. Hierdurch ist verhindert, daß sich die Ausgangsprodukte unterschiedlicher Qualität der beiden Stufen wieder teilweise vermischen. Auch die Gefahr von Rückreaktionen ist hierdurch verringert. Schließlich sei noch erwähnt, daß dem Separator der ersten Stufe Glycerin-Lösung als die abgetrennte schwerere flüssige Phase entnommen wird und zur Weiterverarbeitung bereit steht.

Im folgenden wird ein Prozeß nach dem erfmdungsgemäßen Verfahren mit allen drei Lösungsaspekten anhand der FIGUREN erläutert.

Um die Betriebsphase zu erreichen, muß zunächst eine Startphase gefahren werden, die jedoch noch nicht die optimalen Grenzwerte des erfindungsgemäßen Verfahrens einhält.

### 1.) Startphase

### 1ste Fettspaltung: (mit frischem Enzym)

In einem Reaktor der ersten Stufe werden 30.0 kg rohes, unraffiniertes High-Oleic Sonnenblumenöl 90 plus der Firma Dr. Frische GmbH, (mit einer Säurezahl von 4, ermittelt durch Titration gegen alkalische Kalilauge nach DIN 53169 und DIN 53402), zusammen mit 7.0 kg einer Pufferlösung vorgelegt, die aus einer mit 3.0 g Natriumacetat gepufferten 12 Gew.%igen Glycerin-in-Wasser-Lösung besteht. Die zugeführte Mischung aus Öl und Pufferlösung wird unter Rühren mit der Kreiselpumpe umgewälzt und auf 35-40°C temperiert. Danach werden 30g des Enzyms aus "candida rugosa" (Lipase in Form eines gepulverten Feststoffes von Meito Sangyo, Japan, 360000 U/g) zugegeben.

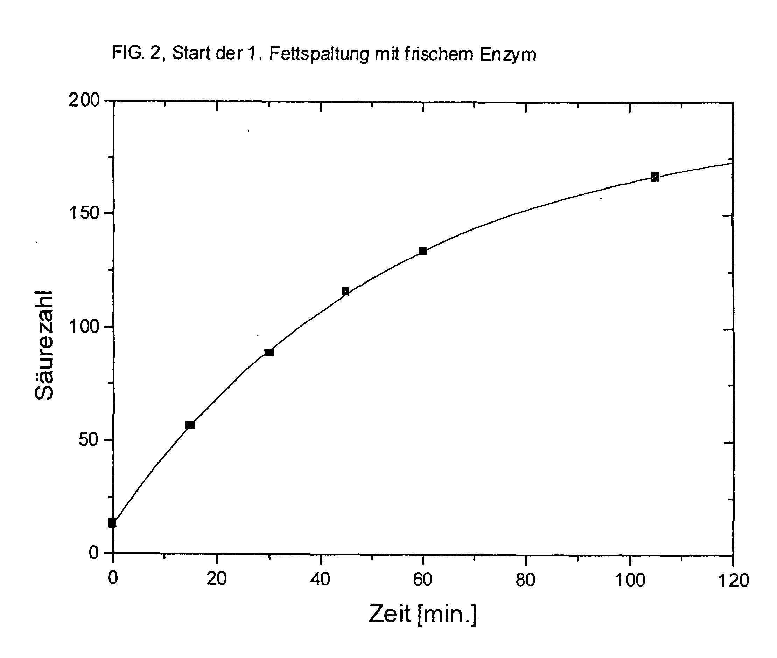

Der Verlauf der Spaltung wird mittels Messung der Säurezahl verfolgt. Nach 50 Minuten beträgt die wie oben durch Titration ermittelte Säurezahl gemäß FIG.2 135, entsprechend einem Spaltgrad von 68%. Der Spaltgrad ergibt sich aus der durch Titration ermittelten Säurezahl (hier 135) dividiert durch die theoretisch berechnete Säurezahl (etwa 199) des aus dem Öl zu erwartenden Gemisches aus freien Fettsäuren und Begleitstoffen. Die Michung wird nun nicht mehr gerührt und nicht mehr umgewälzt. Etwa 5 Minuten, nachdem sowohl der Rührer als auch die Umwälzpumpe abgestellt wurden, wird die beruhigte Mischung im Separator der ersten Stufe (selbstaustragende Teller-Zentrifuge SA1-01 der Firma Westfalia Separator AG) in eine organische, fettsäurehaltige Phase und eine wässrige Glycerin-Phase getrennt, wobei ca. 40 kg/h Reaktorinhalt über die Zentrifuge fließen und die Trommel der Zentrifuge alle 15 Minuten hydraulisch vollständig entleert wird.

Als Entleerungprodukt fallen ca. 2 kg mit Enzym angereicherte Emulsion an. Das Entleerungsprodukt enthält das Enzym, welches sich in der Emulsionsschicht zwischen der organischen Phase und der wässrigen Phase anreichert, und wird in den gerade zu befüllenden Reaktor der ersten Stufe geleitet. Die wässrige Phase ist mit dem aus der Spaltung entstandenen Glycerin auf ca. 36 Gew.% angereichert. Die flüssige organische Phase mit freien Ölsäuren ist nahezu frei von wässriger Phase, enthält nur geringe Restmengen an Enzym und ihre Säurezahl liegt aufgrund der Weiterspaltung während der Phasentrennung beim Abfahren des Behälters über den Separator bei 142.

### 2.) Startphase

### 2te Fettspaltung: (mit frischem Enzym)

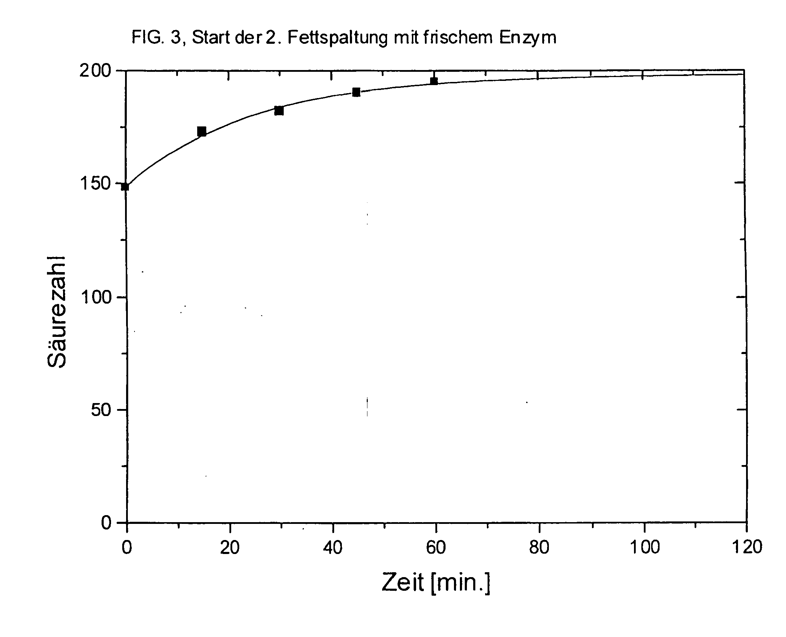

Die ölsäurehaltige organische Phase der ersten Stufe wird in den gerade zu befüllenden Reaktor der zweiten Fettspaltung überführt und hat nun nach Durchmischung am Ende der Nachreaktion eine Säurezahl von 148 (FIG.3). In diesen Reaktor werden 7.0 kg einer frisch angesetzten Pufferlösung aus Weichwasser und 3 g Natriumacetat und 30 g der obigen Lipase gegeben.

Nach 60 min. beträgt die Säurezahl über 190, entsprechend einem Spaltgrad von über 95%. Die Mischung wird nun nicht mehr gerührt und nicht mehr umgewälzt und mit dem Separator der zweiten Stufe unter den selben Bedingungen wie bei der ersten Stufe ausgetragen. Dabei läuft auf der Seite der leichteren Phase eine klare mit Ölsäure angereicherte organische Phase ab. Diese wird der Kurzweg-Destillation unterzogen.

Als schwere Phase wird mit der Zentrifuge eine 12 Gew.%ige Glycerin-in-Wasser/Pufferlösung abgetrennt, die in den gerade zu befüllenden Reaktor der ersten Stufe zurückgeleitet wird. Als Entleerungprodukt fallen ca. 2 kg mit Enzym angereicherte Emulsion an. Diese wird in den zu befüllenden Reaktor der zweiten Stufe geleitet.

### 3.) Betriebsphase

### 1ste Fettspaltung: (mit zurückgewonnenem Enzym)

In einen gerade zu befüllenden Reaktor der ersten Stufe werden 30,0 kg des obigen Öls zusammen mit der 12 Gew.%igen Glycerin-in-Wasser/Pufferlösung aus der zweiten Separatorstufe (hier noch aus der Startphase) vorgelegt. Diese Mischung wird unter Rühren mit einer Kreiselpumpe umgewälzt und auf 35-40°C temperiert. Danach werden die 2 kg Entleerungprodukt aus der ersten Separatorstufe des "Startversuchs", die mit Enzym angereicherte Emulsion und 3g frische Lipase des obigen Typs nachdosiert.

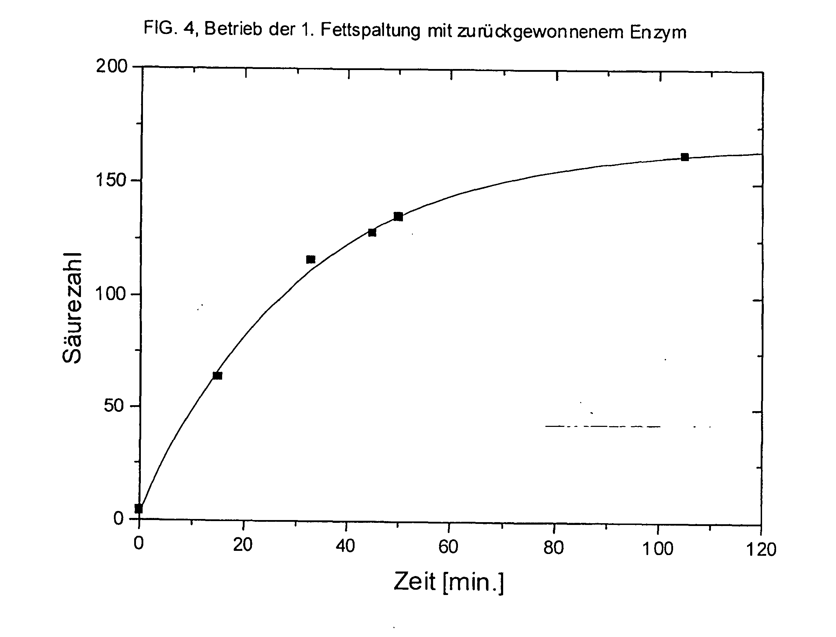

Nach 60 min. beträgt die Säurezahl 134 (FIG.4), entsprechend einem Spaltgrad von ca. 68%. Nun wird die Mischung wie in der Startphase getrennt, wobei auf der Seite der schweren Phase nun eine ca. 36 Gew.%ige Glycerin-in-Wasser-Lösung abfließt und aus dem Prozeß entnommen wird.

### 4.) Betriebsphase

### 2te Fettspaltung: (mit zurückgewonnenem Enzym)

Der zu befüllende Reaktor der zweiten Stufe erhält die ölsäurehaltige organische Phase der ersten Stufe und hat nach der Durchmischung des Reaktorinhalts eine Säurezahl von 142. Ferner werden in den Reaktor 7,0 kg der wie oben beschriebenen Pufferlösung gegeben sowie die 2 kg Entleerungprodukt aus der zweiten Separatorstufe des "Startversuchs", nämlich die mit Enzym angereicherte Emulsion, und 5g frische Lipase des obigen Typs zur Nachschärfüng.

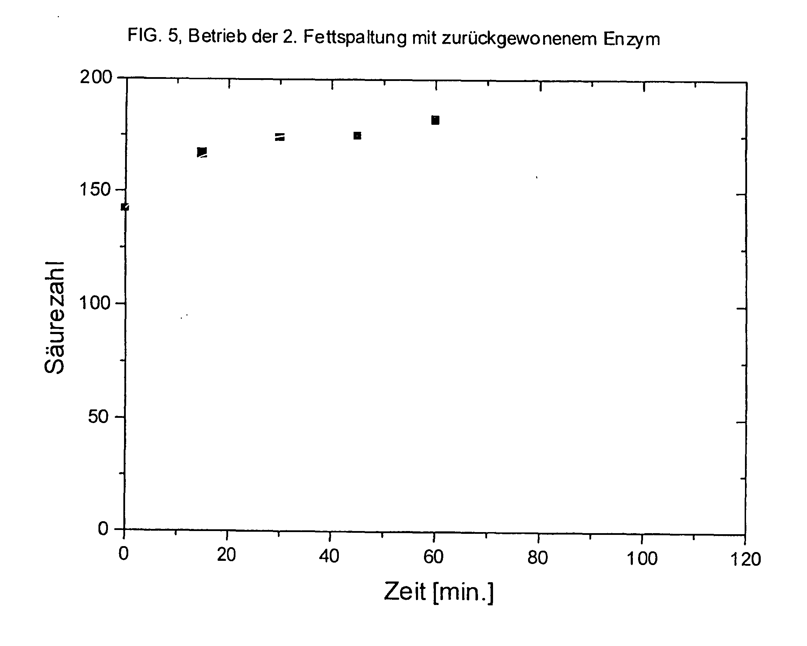

Nach 60 min. beträgt die Säurezahl 184 (FIG.5), entsprechend einem Spaltgrad von 93%. Die Ausschleusung der Reaktionsprodukte erfolgt wie in der Startphase, wobei die ablaufende klare Ölsäure-angereicherte organische Phase eine Säurezahl von 185 aufweist. Als schwere Phase wird mit der Zentrifuge eine 12 Gew.%ige Glycerin-Wasser/Pufferlösung abgetrennt. Als Entleerungprodukt fallen ca. 2 kg mit Enzym angereicherte Emulsion an.

### 5.) Kurzweg-Destillation/Entwachsung:

Die so in der zweiten Stufe gewonnene Roh-Fettsäure (Säurezahl 184) wird in einem als Zwischenbehälter dienenden 200 1-Faß gesammelt und einer Kurzwegdestillation auf einer Vakuum-Kurzwegdestille vom Typ KD 10 der Firma UIC, Alzenau-Hörstein, mit vorgeschalteter Entgasungsstufe zur Abtrennung etwaiger geringer Wassermengen unterzogen. Beispielsweise wird dann bei einem Gesamtdruck von 0,014 mbar und 191 °C destilliert, wobei ca. 8 Gew.% der eingesetzten Fettsäure kontinuierlich als Rückstandsprodukt auf der Seite der hierbei nicht siedenden Bestandteile ablaufen. Die anderen 92 Gew.% des Rohproduktes destillieren als Ölsäure mit einer Säurezahl von 199-200 und nur geringsten Mengen an Begleitstoffen in Form von Fettsäuren mit niedrigerem Dampfdruck. Dies entspricht im Rahmen der Meßgenauigkeit der theoretisch berechneten Säurezahl. (Der Wert ist aufgrund des geringen Anteils an leichter flüchtigen kürzeren Fettsäuren etwas höher als die theoretische Säurezahl).

Das Rückstandsprodukt wird einer Entwachsung unterzogen, wobei die enthaltenen höheren Fettsäuren ( C22 und größere Kettenlängen ), Wachse und andere höher siedenden Begleitstoffe des Öls als Feststoffe aus kristallisieren und mittels Filtration abgetrennt werden.

### 6.) Wiedereinsatz des Rücköls

Der Wiedereinsatz des Rücköls oder Sumpfes wurde zur Demonstration des Verfahrensvorteils als Aliquot im Labormaßstab durchgeführt. Dies war auch gerechtfertigt, da aus den Vorversuchen bekannt war, daß im Labor sehr gut vergleichbare Bedingungen zur enzymatisch katalysierten Umsetzung des Öls mit Wasser einstellbar sind.

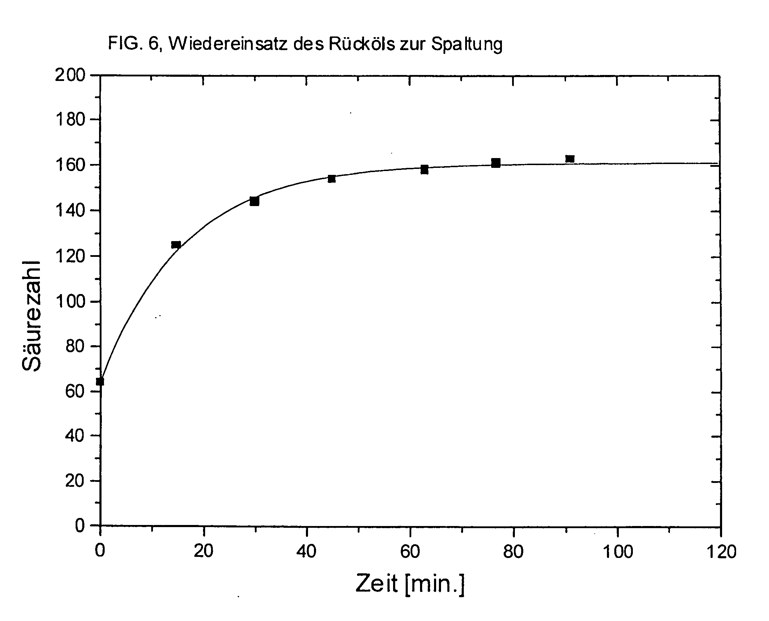

In einen 500ml Rundkolben werden 91.5g des filtrierten Rückstandsproduktes zusammen mit 91.5g rohem, unraffiniertem High-Oleic Sonnenblumenöl 90 plus der Dr. Frische GmbH, und 46.0g der gepufferten 12 Gew.%igen Glycerin-in-Wasser Lösung aus der zweiten Separator-Stufe angesetzt und mit einem Magnetrührer bei 1000 U/min durchmischt. Die Säurezahl der Mischung beträgt 64. Dabei wird die Reaktionsmischung auf dem Wasserbad auf ca. 40°C temperiert. Nachdem die Reaktionsmischung auf 40°C erwärmt ist, wird die zu obigem Versuch adäquate Menge von 0.19g an Lipase obigen Typs zugegeben und die Reaktion anhand der Säurezahl verfolgt (siehe FIG.6). Der Säurezahlverlauf zeigt, daß die Rückführung des Rücköls den Prozeß hinsichtlich der Wirtschaftlichkeit verbessert.

Im Ausführungsbeispiel wurde im Spaltreaktor mit einer Temperatur von 35 bis 40 °C gearbeitet. Das erfindungsgemäße Verfahren läßt sich jedoch grundsätzlich zwischen etwa 20 °C und 90°C durchführen. Die untere Grenze wird dabei durch die Fließfähigkeit der zu durchmischenden Flüssigkeit bestimmt, während die obere Grenze durch die thermische Stabilität des Enzyms und durch die niedrigste Siedetemperatur im Flüssigkeitsgemisch begrenzt wird.

Die Anzahl der Spalt-Stufen kann erhöht werden, wobei z.B. schon bei drei Stufen die Glycerinkonzentration spürbar erhöht werden kann.

Wie bereits erwähnt, kann man nach dem zweiten und dritten Lösungsaspekt der vorliegenden Erfindung bereits nach kurzer Zeit auf beachtlich hohe Spaltgrade kommen, die, wie die Startphase des Technikumsversuchs zeigt, auch über 95% erreichen. Hierbei ist allerdings der Verfahrensweg nach Anspruch 1 und 2 erschwert.

Der im einzigen oder letzten Spaltreaktor erzielte Spaltgrad kann in weiten Grenzen variiert werden. Empfehlenswert sind Werte nicht unter 80% und bevorzugt Werte über 90%, um die Verfahrenskapazitäten voll ausschöpfen zu können.

Bei Spezialanwendungen beispielsweise mit spezifischen Enzymen kann man auch unter einem Spaltgrad von 60% arbeiten.

Die erfindungsgemäße Enzymspaltung wurde auch erfolgreich auf Fettsäuremethylester angewandt. Grundsätzlich lassen sich enzymatisch spaltbare Ester mit Vorteil nach dem zweiten und dritten Aspekt der Erfindung spalten, wobei die Anwendungsmöglichkeit des Verfahrens nach dem ersten Lösungsaspekt von den entstehenden Alkohol- und Fettsäurekomponenten abhängt.

## Patentansprüche

1. Verfahren zur enzymatischen Spaltung von Ölen und Fetten zum Gewinnen von Fettsäuren und Glycerin unter Einsatz von als Biokatalysatoren wirkenden Lipasen auf ein Gemisch, das ein Öl oder Fett und Wasser enthält,
**dadurch gekennzeichnet,**
**dass** die Spaltreaktion nur bis zu einem Spaltgrad zwischen 60 und 95 % durchgeführt wird, bei dem für das eingesetzte Gemisch die Verlangsamung der Spaltreaktion unter einem vorgegebenen Wert liegt, dass die zu gewinnenden Fettsäuren vom nur teilweise gespaltenen Reaktionsgemisch abgetrennt werden, indem zunächst die sich beim Spaltprozess bildende wässrige, glycerinhaltige Phase von der teilgespaltenen organischen Phase mit den abgespaltenen Fettsäuren getrennt wird und anschließend die Fettsäuren von der teilgespaltenen organischen Phase abgetrennt werden, und dass der von den freien Fettsäuren befreite verbleibende Rest der organischen Phase in den Spaltprozess zurückgeführt wird.

2. Verfahren nach Anspruch 1,
**dadurch gekennzeichnet,**
**dass** zur Abtrennung der Fettsäuren eine Vacuumdestillation, vorzugsweise eine Kurzweg-Destillation oder Fallfilm-Destillation, durchgeführt wird.

3. Verfahren gemäß Anspruch 1,
**dadurch gekennzeichnet, dass** das bei der Spaltung gebildete Reaktionsgemisch zur Trennung der sich beim Spaltprozess bildenden wässrigen, glycerinhaltigen Phase von der organischen Phase, die abgespaltene freie Fettsäuren enthält, in einen selbstaustragenden Separator gegeben wird, der so eingestellt wird, dass die sich zwischen der wässrigen und organischen Phase bildende, mit Lipase angereicherte Zwischenschicht sich im Tellerpaket des Separators ansammelt, dass der Separator zu vorgegebenen Zeitpunkten entleert wird und dass der dabei ausgetragene Trommelinhalt des Separators erneut der Fettspaltung zugeführt wird.

4. Verfahren gemäß Anspruch 1,
**dadurch gekennzeichnet, dass** die Fettspaltung diskontinuierlich in Spaltreaktoren durchgeführt wird, die im Schlaufenbetrieb mit durch Pumpen umgewälztem Reaktorinhalt betrieben werden, wobei mehrere Reaktoren parallel für eine einzige oder eine mehrerer Fettspaltungsstufen vorgesehen sind, einer der Reaktoren bei nicht aktivierter Umwälzschlaufe mit Öl bzw. Fett befüllt wird, währenddessen ein weiterer Reaktor mit aktiver Umwälzschlaufe im Spaltbetrieb gefahren wird und ein noch weiterer Reaktor ebenfalls bei nicht aktivierter Umwälzschlaufe über einen Separator entleert wird, der die sich beim Spaltprozess bildende wässrige, glycerinhaltige Phase von der organischen Phase, die abgespaltene freie Fettsäuren enthält, trennt, bevor die Fettsäuren von der organischen Phase getrennt werden.

5. Verfahren nach einem der Ansprüche 1,2 oder 4,
**dadurch gekennzeichnet,**
**dass** das bei der Spaltung gebildete Reaktionsgemisch zur Trennung der sich beim Spaltprozess bildenden wässrigen, glycerinhaltigen Phase von der organischen Phase, die abgespaltene freie Fettsäuren enthält, in einen selbstaustragenden Separator gegeben wird, der so eingestellt wird, dass die sich zwischen der wässrigen und organischen Phase bildende, mit Lipase angereicherte Zwischenschicht sich im Tellerpaket des Separators ansammelt, dass der Separator zu vorgegebenen Zeitpunkten entleert wird und dass der dabei ausgetragene Separatorinhalt erneut der Fettspaltung zugeführt wird.

6. Verfahren nach einem der Ansprüche 1, 2, 3 oder 5,
dass die Fettspaltung diskontinuierlich in Spaltreaktoren durchgeführt wird, die im Schlaufenbetrieb mit durch Pumpen umgewälztem Reaktorinhalt betrieben werden, wobei mehrere Reaktoren parallel für eine einzige oder eine mehrerer Fettspaltungsstufen vorgesehen sind, einer der Reaktoren bei nicht aktivierter Umwälzschlaufe mit Öl bzw. Fett befüllt wird, währenddessen ein weiterer Reaktor mit aktiver Umwälzschlaufe im Spaltbetrieb gefahren wird und ein noch weiterer Reaktor ebenfalls bei nicht aktivierter Umwälzschlaufe über einen Separator entleert wird, der die sich beim Spaltprozess bildende wässrige, glycerinhaltige Phase von der organischen Phase, die abgespaltene freie Fettsäuren enthält, trennt, bevor die Fettsäuren von der organischen Phase getrennt werden.

7. Verfahren nach einem der vorhergehenden Ansprüche,
**dadurch gekennzeichnet,**
**dass** als Lipasen unspezifische Lipasen, spezifische Lipasen oder Mischungen von unspezifischen und spezifischen Lipasen eingesetzt werden.

8. Verfahren nach einem der vorhergehenden Ansprüche,
**dadurch gekennzeichnet,**
**dass** der bei der Abtrennung der Fettsäuren von der gewonnenen organischen Phase verbleibende Öl- bzw. Fettrückstand von Wachsen befreit wird und anschließend dem Spaltprozess wieder zugeführt wird.

9. Verfahren nach einem der vorhergehenden Ansprüche,
**dadurch gekennzeichnet,**
**dass** die Fettspaltung in zwei Stufen erfolgt, in denen jeweils ein Teil des gewünschten Spaltgrades erbracht wird, wobei die in der zweiten Stufe gewonnene wässrige Glycerinphase der ersten Stufe als Wasserphase zugeführt wird und der zweiten Stufe als Wasserphase Frischwasser und ferner die in der ersten Stufe gewonnene organische Phase zugeführt werden.

10. Verfahren nach Anspruch 9 in Verbindung mit Anspruch 1,
**dadurch gekennzeichnet,**
**dass** der verbleibende Rest der organischen Phase der ersten Stufe der Fettspaltung zugeführt wird.

11. Verfahren nach Anspruch 1 und nach einem der auf Anspruch 1 rückbezogenen Ansprüche,
**dadurch gekennzeichnet,**
der Rest der organischen Phase, vorzugsweise nach Durchlaufen einer Entwachsungsstation, mit frischem zu spaltendem Gemisch, welches Öl bzw. Fett, Wasser und Lipase enthält, vermischt wird.

12. Verfahren nach Anspruch 3 und einem der auf Anspruch 3 rückbezogenen Ansprüche,
**dadurch gekennzeichnet,**
**dass** die aus dem selbstaustragenden Separator abfließende organische Phase einem weiteren selbstaustragenden Separator in Form einer Polierzentrifuge zugeführt wird, die ebenfalls intermittierend entleert wird, um die sich als Sediment an der Zentrifugenwand abscheidenden Enzymrestmengen für den Spaltprozess wieder zu gewinnen.

13. Verfahren nach einem der vorhergehenden Ansprüche, angewendet auf die Spaltung der Mono-, Di- und Triglyceride von Soap-Stocks.

## Claims

1. Process for the enzymatic hydrolysis of oils and fats to obtain fatty acids and glycerol by using lipases acting as biocatalysts on a mixture that contains an oil or fat and water,
**characterized in that** the hydrolysis reaction is carried out only up to a reaction rate between 60 and 95%, at which, for the mixture used, the retardation of the hydrolysis reaction is below a predefined value, **in that** the fatty acids to be obtained are separated from the only partly hydrolysed reaction mixture by, firstly, the aqueous, glycerol-containing phase forming during the hydrolysis phase being separated from the partly hydrolysed organic phase with the hydrolysed fatty acids and then the fatty acids being separated from the partly hydrolysed organic phase, and **in that** the remaining residue of the organic phase freed of the fatty acids is fed back into the hydrolysis process.

2. Process according to Claim 1, **characterized in that**, in order to separate the fatty acids, vacuum distillation, preferably short-path distillation or falling-film distillation, is carried out.

3. Process according to Claim 1, **characterized in that**, in order to separate the aqueous, glycerol-containing phase formed during the hydrolysis process from the organic phase, which contains hydrolysed free fatty acids, the reaction mixture formed during the hydrolysis is put into a self-discharging separator, which is set in such a way that the lipase-enriched intermediate layer forming between the aqueous and organic phase accumulates in the plate stack of the separator, **in that** the separator is emptied at predefined times, and **in that** the drum contents of the separator discharged in the process are fed back to the fat hydrolysis again.

4. Process according to Claim 1, **characterized in that** the fat hydrolysis is carried out discontinuously in hydrolysis reactors which are operated in loop operation with reactor contents circulated by pumps, a plurality of reactors being provided in parallel for a single fat hydrolysis stage or one of a plurality of fat hydrolysis stages, one of the reactors being filled with oil or fat with the circulation loop not activated, while a further reactor is run in hydrolysis operation with the circulation loop active, and yet another reactor being emptied, with the circulation loop likewise not activated, via a separator which separates the aqueous, glycerol-containing phase formed during the hydrolysis process from the organic phase, which contains hydrolysed free fatty acids, before the fatty acids are separated from the organic phase.

5. Process according to one of Claims 1, 2 or 4, **characterized in that**, in order to separate the aqueous, glycerol-containing phase formed during the hydrolysis process from the organic phase, which contains hydrolysed free fatty acids, the reaction mixture formed during the hydrolysis is put into a self-discharging separator, which is set in such a way that the lipase-enriched intermediate layer forming between the aqueous and organic phase accumulates in the plate stack of the separator, **in that** the separator is emptied at predefined times, and **in that** the separator contents discharged in the process are fed back to the fat hydrolysis again.

6. Process according to Claim 1, 2, 3 or 5, **characterized in that** the fat hydrolysis is carried out discontinuously in hydrolysis reactors which are operated in loop operation with reactor contents circulated by pumps, a plurality of reactors being provided in parallel for a single fat hydrolysis stage or one of a plurality of fat hydrolysis stages, one of the reactors being filled with oil or fat with the circulation loop not activated, while a further reactor is run in hydrolysis operation with the circulation loop active, and yet another reactor being emptied, with the circulation loop likewise not activated, via a separator which separates the aqueous, glycerol-containing phase formed during the hydrolysis process from the organic phase, which contains hydrolysed free fatty acids, before the fatty acids are separated from the organic phase.

7. Process according to one of the preceding claims, **characterized in that** the lipases used are non-specific lipases, specific lipases or mixtures of non-specific and specific lipases.

8. Process according to one of the preceding claims, **characterized in that** the oil or fat residue remaining during the separation of the fatty acids from the organic phase obtained is freed of waxes and then fed to the hydrolysis process again.

9. Process according to one of the preceding claims, **characterized in that** the fat hydrolysis is carried out in two stages, in which in each case a part of the desired reaction rate is produced, the aqueous glycerol phase obtained in the second stage being fed to the first stage as aqueous phase, and the second stage being fed with fresh water as aqueous phase and also with the organic phase obtained in the first stage.

10. Process according to Claim 9 in conjunction with Claim 1, **characterized in that** the remaining residue of the organic phase is fed to the first stage of the fat hydrolysis.

11. Process according to Claim 1 and according to one of the claims referring back to Claim 1, **characterized in that** the remainder of the organic phase, preferably after passing through a de-waxing station, is mixed with fresh mixture to be hydrolysed, which contains oil or fat, water and lipase.

12. Process according to Claim 3 and one of the claims referring back to Claim 3, **characterized in that** the organic phase flowing out of the self-discharging separator is fed to a further self-discharging separator in the form of a polishing centrifuge, which is likewise emptied intermittently in order to recover the residual quantities of enzyme which have been deposited as a sediment on the centrifuge wall for reuse in the hydrolysis process.

13. Process according to one of the preceding claims, applied to the hydrolysis of the mono-, di- and triglycerides of soap stocks

## Revendications

1. Procédé pour la dissociation enzymatique d'huiles et de graisses pour l'obtention d'acides gras et de glycérine, recourant à des lipases agissant comme des biocatalyseurs dans un mélange contenant une huile ou de la graisse et de l'eau,
**caractérisé en ce que** la réaction de dissociation n'est appliquée que jusqu'à un taux de dissociation compris entre 60 et 95 % où le ralentissement de la réaction de dissociation est inférieur à une valeur prédéfinie pour le mélange mis en oeuvre, **en ce que** les acides gras à obtenir sont séparés du mélange réactif qui n'est que partiellement dissocié, la phase aqueuse, à teneur en glycérine formée lors du processus de dissociation étant d'abord séparée de la phase organique partiellement dissociée avec les acides gras dissociés, puis les acides gras séparés de la phase organique partiellement dissociée, et **en ce que** le reste de la phase organique débarrassé des acides gras libres est reconduit vers le processus de dissociation.

2. Procédé selon la revendication 1, **caractérisé en ce que** pour la séparation des acides gras, il est procédé à une distillation sous vide, de préférence une distillation à évaporation instantanée ou une distillation à film descendant.

3. Procédé selon la revendication 1, **caractérisé en ce que**, pour la séparation de la phase aqueuse à teneur en glycérine formée lors du processus de dissociation de la phase organique contenant des acides gras libres dissociés, le mélange réactif formé lors de la dissociation est ajouté dans un séparateur à auto-évacuation réglé de telle manière que la couche intercalaire enrichie en lipase formée entre la phase aqueuse et la phase organique se dépose dans le bol à assiettes du séparateur, que le séparateur soit vidangé à des moments définis et que le contenu de tambour alors évacué du séparateur soit reconduit vers la dissociation lipidique.

4. Procédé selon la revendication 1, **caractérisé en ce que** la dissociation lipidique est exécutée de manière discontinue dans des réacteurs de dissociation mis en service en mode d'écoulement en boucles avec un contenu de réacteur recyclé par des pompes, plusieurs réacteurs étant prévus en parallèle pour un seul ou pour plusieurs étages de dissociation lipidique, un des réacteurs étant rempli d'huile ou de graisse lorsque la boucle de circulation est désactivée, tandis qu'un autre réacteur est lancé en mode de dissociation avec une boucle de circulation activée, et qu'un troisième réacteur est également vidangé avec une boucle de circulation désactivée sur un séparateur séparant la phase aqueuse à teneur en glycérine formée lors du processus de dissociation de la phase organique contenant des acides gras libres dissociés, avant que les acides gras soient séparés de la phase organique.

5. Procédé selon l'une des revendications 1, 2 ou 4, **caractérisé en ce que**, pour la séparation de la phase aqueuse à teneur en glycérine formée lors du processus de dissociation de la phase organique contenant des acides gras libres dissociés, le mélange réactif formé lors de la dissociation est ajouté dans un séparateur à auto-évacuation réglé de telle manière que la couche intercalaire enrichie en lipase formée entre la phase aqueuse et la phase organique se dépose dans le bol à assiettes du séparateur, que le séparateur soit vidangé à des moments définis et que le contenu de séparateur alors évacué soit reconduit vers la dissociation lipidique.

6. Procédé selon l'une des revendications 1, 2, 3 ou 5, **caractérisé en ce que** la dissociation lipidique est exécutée de manière discontinue dans des réacteurs de dissociation mis en service en mode d'écoulement en boucles avec un contenu de réacteur recyclé par des pompes, plusieurs réacteurs étant prévus en parallèle pour un seul ou pour plusieurs étages de dissociation lipidique, un des réacteurs étant rempli d'huile ou de graisse lorsque la boucle de circulation est désactivée, tandis qu'un autre réacteur est lancé en mode de dissociation avec une boucle de circulation activée, et qu'un troisième réacteur est également vidangé avec une boucle de circulation désactivée sur un séparateur séparant la phase aqueuse à teneur en glycérine formée lors du processus de dissociation de la phase organique contenant des acides gras libres dissociés, avant que les acides gras soient séparés de la phase organique.

7. Procédé selon l'une des revendications précédentes, **caractérisé en ce que** les lipases mises en oeuvre sont des lipases non spécifiques, des lipases spécifiques ou des mélanges de lipases non spécifiques et de lipases spécifiques.

8. Procédé selon l'une des revendications précédentes, **caractérisé en ce que** le résidu d'huile ou de graisse restant lors de la séparation des acides gras de la phase organique obtenue est débarrassé des cires avant d'être reconduit vers le processus de dissociation.

9. Procédé selon l'une des revendications précédentes, **caractérisé en ce que** la dissociation est exécutée sur deux étages, dans chacun desquels une partie du taux de dissociation souhaité est obtenue, la phase aqueuse glycérinique obtenue dans le deuxième étage étant conduite vers le premier étage en tant que phase aqueuse, et de l'eau fraîche ainsi que la phase organique obtenue dans le premier étage étant conduites vers le deuxième étage en tant que phase aqueuse.

10. Procédé selon la revendication 9 en relation avec la revendication 1, **caractérisé en ce que** le reste de la phase organique est conduit vers le premier étage de la dissociation lipidique.

11. Procédé selon la revendication 1 et selon l'une des revendications dépendantes de la revendication 1, **caractérisé en ce que** le reste de la phase organique est mélangé à un mélange frais à dissocier contenant une huile ou de la graisse de l'eau et une lipase, de préférence après passage par une station de décirage.

12. Procédé selon la revendication 3 et selon l'une des revendications dépendantes de la revendication 3, **caractérisé en ce que** la phase organique s'écoulant du séparateur à auto-évacuation est conduite vers un autre séparateur à auto-évacuation sous la forme d'une centrifugeuse de polissage également à vidanges intermittentes pour récupérer pour le processus de dissociation les quantités enzymatiques résiduelles séparées comme sédiment contre la paroi de centrifugeuse.

13. Procédé selon l'une des revendications précédentes, utilisé pour la dissociation des monoglycérides, diglycérides et triglycérides de pâtes de neutralisation.
